# EUROPEAN PATENT SPECIFICATION

(11) **EP 3 560 159 B1**
(45) Date of publication and mention of the grant of the patent: **29.11.2023**
(21) Application number: 17829636.4
(22) Date of filing: 21.12.2017
(51) Int. Cl.: G16H 80/00, G16H 40/67, H04W 28/22

(54) **TRANSMISSION BANDWIDTH REQUESTING MOBILE MEDICAL DEVICE**
ÜBERTRAGUNGSBANDBREITENANFORDERNDE MOBILE MEDIZINISCHE VORRICHTUNG
DISPOSITIF MÉDICAL MOBILE NÉCESSITANT UNE BANDE PASSANTE DE TRANSMISSION

(30) Priority: 22.12.2016 EP 16206064
(43) Date of publication of application: 30.10.2019
(73) Proprietor: Koninklijke Philips N.V., 5656 AG Eindhoven (NL)
(72) Inventor: VOGTMEIER, Gereon, 5656 AE Eindhoven (NL); DOKANIA, Anand, Kumar, 5656 AE Eindhoven (NL)
(74) Representative: Philips Intellectual Property & Standards
(86) International application number: PCT/EP2017/083964
(87) International publication number: WO 2018/115214

(56) References cited:
- EP-A1- 1 788 501
- WO-A1-2014/205098
- US-A1- 2004 049 233

## Description

### FIELD OF THE INVENTION

The invention relates to a medical device, a method, and a program element for facilitating remote medical support.

### BACKGROUND OF THE INVENTION

Mobility is an important aspect for many medical devices. Traditionally, patients have to visit a hospital or other medical centers for medical treatments or examinations to be performed. This imposes a major burden on patients, in particular in rural areas, where distances to the next hospital can be long. The requirement for patients to reach a hospital can result in critical delays especially in emergency situations. Moreover, substantial costs arise for hospitals and the healthcare system in general when patients have to reside within a hospital for a long period of time due to the availability of certain medical devices only inside the hospital.

At the same time, medical devices are becoming increasingly sophisticated, and the knowledge to properly operate such devices is becoming more and more evolved. Moreover, the operators of mobile medical devices are often less specialized as compared to experts working in hospitals. Instead, operators of mobile medical devices may be faced with a broad spectrum of tasks and they may have to handle a variety of different medical devices. For these reasons, the operators of mobile medical devices may not have the experience to fully understand all use cases, configuration settings, and results of the medical devices. For example, an operator of an X-ray or ultrasound imaging device may be experienced with the basic operation of the device, but may have a lack of experience in analyzing the captured images.

To support operators of medical devices, especially mobile medical devices, communication systems can be integrated into such systems, which allow the operator to exchange information with remote medical experts. In case of mobile medical devices, these communication systems often utilize wireless technologies. However, the achievable transmission rates with wireless technologies vary widely depending on numerous parameters such as the communication technology, the sharing of communication resources with other users as well as characteristics of the wireless channel such as slow and fast fading, multipath effects, Doppler shifts, etc. As a result, traditional communication systems often do not provide sufficient transmission rates to enable efficient support of the operator of a medical device by a remote medical expert.

Document EP1788501 A1, which represents the closest prior art, discloses a medical device with selectable, different status monitoring modes, wherein a data transmission mode for transmitting data sets is determined and a corresponding communication connection is established.

Document US2012253847 discloses a medical device for facilitating remote medical support, wherein communications quality of service parameters are controlled for medical examinations with varying urgency.

### SUMMARY OF THE INVENTION

The invention is set out in the appended claims.

### SUMMARY OF THE DISCLOSURE

The inventors of the present invention have found that for medical devices, in particular mobile medical devices, there is a need to improve the communication of the operator of the medical device with a remote medical expert.

The object of the present invention may be seen as to facilitate a more efficient exchange of information between the operator of a medical device and a remote medical expert.

The problem of the present invention is solved by the subject-matter of the independent claims, wherein further embodiments are incorporated in the dependent claims.

The described embodiments similarly pertain to the medical device for facilitating remote medical support, the method for facilitating remote medical support, and the program element for facilitating remote medical support. Synergetic effects may arise from different combinations of the embodiments although they might not be described in detail.

Further on, it shall be noted that all embodiments of the present invention concerning a method might be carried out with the order of the steps as described. Nevertheless, this has not to be the only and essential order of the steps of the method. The methods presented herein can be carried out with another order of the disclosed steps without departing from the respective method embodiment, unless explicitly mentioned to the contrary hereinafter.

Technical terms are used by their common sense. If a specific meaning is conveyed to certain terms, definitions of terms will be given in the following in the context of which the terms are used.

According to the present invention, a medical device for facilitating remote medical support according to claims 1-9 is presented.

Any of the following subject-matter described as embodiments which does not fall within the scope of the claims are to be considered as illustrative only.

The medical device comprises a user interface and a communication unit, wherein the user interface is configured for querying and receiving an input regarding at least one current medical use condition of the medical device. In addition, the communication unit is configured for determining a required transmission rate for transmitting data from the medical device to a remote medical expert, where the required transmission rate is determined based on the received input regarding the current medical use condition of the medical device. Moreover, the communication unit is configured for requesting and establishing one or more communication connections to achieve the required transmission rate.

The user interface is configured for querying information regarding current medical use conditions of the medical device from the operator. Examples of such medical use conditions can be, amongst others, the medical experience level of the operator, the experience level of the operator with the specific medical device, the urgency level or the current use case of the medical device. The use case can be, amongst others, a diagnostic task or medical treatment to be performed. For instance, the user interface of an X-ray imaging device may query information about the object to be imaged (human, animal, age, gender, etc.) and the region of interest to be investigated. Furthermore, the user interface of an X-ray imaging device may be configured for querying acquisition scan parameters, whether or not a sequence of images is to be taken, the number of images, the number of patients, the resolution of X-ray images as well as other information that is necessary to estimate the amount of data to be transmitted to a remote medical expert.

The user interface may adapt to the information provided by the operator of the medical device. That means, the user interface may be configured for adjusting for instance windows, tabs, menus, icons and control elements depending on input regarding current medical use conditions of the medical device. For example, if the operator inputs that he has little experience with the medical device, then fixed default values may be chosen for some parameters, and the user interface may hide these parameters. Hence, an unexperienced operator may be presented a simplified variant of the user interface to ease the usage of the medical device. The user interface is configured for receiving the information about the current medical use condition from the operator and for providing this information to the communication unit of the medical device.

Subsequently, the medical device may determine the data that needs to be sent to a remote medical expert in order to support the operator of the medical device. Thereto, the medical device may utilize the information about the medical use condition provided by the user interface. Note that throughout this document, when referring to a remote medical expert, this encompasses a remote human expert or alternatively an expert system running on a remote server. As another alternative, the expression remote medical expert may mean a remote human interacting with a medical expert system.

The amount of information that needs to be sent to the remote medical expert notably depends on the medical experience level of the operator of the medical device. For example, a well-experienced operator of an X-ray or ultrasound imaging device may require little support from a remote medical expert, and the remote medical expert may be able to provide this support based on a small selected amount of data. At the same time, an unexperienced operator of the imaging device may require extensive support from a remote medical expert. Thereto, it may be necessary to send large amounts of data comprising for instance a sequence of high resolution images to the remote medical expert. The medical device may be configured for utilizing data compression techniques for reducing the amount of data that needs to be transmitted to the remote medical expert. Data compression techniques may be particularly beneficial for reducing the amount of data of ultrasound videos or computer tomography data. Depending on the amount of data that needs to be sent to the remote medical expert and the urgency level, the communication unit then determines a required transmission rate to facilitate the efficient exchange of information between the operator of the medical device and the remote medical expert.

The communication unit is further configured for requesting and establishing one or more communication connections to meet the required transmission rate. Thus, the communication unit is adapted to support several communication connections. For example, the communication unit may be able to establish one or more connections over cellular communication networks such as GSM, UMTS or LTE connections. The transmission rates achievable by means of such wireless connections strongly depend on a number of parameters such as the communication technology, network congestion as well as characteristics of the wireless channel such as slow and fast fading, multipath effects, Doppler shifts, etc. As a result, a single cellular communication connection may not suffice to meet the required transmission rate. In this case, the communication unit may automatically request one or more other connections, which can potentially be of other types. For example, the communication unit may request a Wi-Fi connection, or it may be possible to utilize wireline connections using Ethernet, amongst others. In addition, in some scenarios, it may be possible to request the starting of communication balloons or drones to establish a data repeater infrastructure. In general, the availability of communication technologies depends on the location. Thus, the communication unit has to meet the required transmission rate using locally available communication technologies. The combination of communication connections can allow the usage of the mobile medical device in areas where a single connection would not be sufficient to meet the required transmission rate.

Preferably, the communication unit may be configured for ensuring that also the transmission rate in the reverse direction from the remote expert to the mobile medical device is sufficient to enable efficient communications bidirectionally between the operator of the mobile medical device and the remote medical expert. Thereto, the required transmission rates have to be determined for the link from the medical device to the remote medical expert and, in addition, for the link from the remote medical expert to the medical device. Moreover, the communication unit has to be configured for requesting and establishing communication connections so that the required transmission rates are achieved for both links between the medical device and the remote medical expert.

In an exemplary embodiment, the user interface of the medical device is configured for querying the input of a medical experience level of a current operator of the medical device, and/or the input of a current medical task.

The input of the medical experience level of the operator is used to classify the operator according to his medical experience level and to assess the support level that is needed for the operator. In particular, the user interface may be configured for adapting its design including for instance windows, tabs, menus, icons and control elements to the medical experience level of the operator. In addition, the data set to be transmitted to a remote medical expert depends on the medical experience level of the operator. For example, a well-experienced operator may require little support from a remote expert, and this support may be realizable by transmitting a small amount of selected data to the remote expert. In contrast, an unexperienced operator may require substantial support from a remote expert. In this case, it may be necessary to transmit large amounts of data to the remote expert. For example, for an X-ray or ultrasound imaging device, an unexperienced operator may have to transmit a sequence of high-resolution images to the remote expert.

The input of the current medical task determines for example a diagnostic task to be solved by means of the medical device. For example, for an X-ray imaging device, the input of the current medical task may include information about the object and the region of interest to be imaged. If for instance an X-ray image of an arm of an adult is to be taken, then a moderately experienced operator of the X-ray imaging device may be able to analyze the image with little support from a remote expert. In contrast, the operator may not be experienced in analyzing X-ray images of the brain. For this reason, the operator may prefer to transmit all captured data to a remote medical expert in case an X-ray image of a brain is to be taken and analyzed. Hence, the data that needs to be sent to a remote medical expert also depends on the task to be solved by means of the medical device.

In another exemplary embodiment, the medical device is configured for determining a data set that needs to be communicated to the remote medical expert based on the received input regarding the current medical use condition of the medical device. Furthermore, the communication unit is configured for determining a required transmission rate for transmitting the determined data set to the remote medical expert.

As noted above, the input regarding the current medical use condition of the medical device may comprise input regarding the medical experience level of the operator, the experience level of the operator with the specific medical device, the urgency level and the use case of the medical device. Depending on the input regarding the medical experience level of the operator, the experience level with the medical device and the use case of the medical device, the support level is determined by the medical device. That means, the medical device is configured for assessing the amount of support that the operator requires from the remote medical expert to perform the medical task. Based on this information, the communication unit may determine the data set that needs to be communicated to the remote medical expert. For example, in case of an X-ray imaging device, an experienced operator may need to send only a selected region of an X-ray image to the remote medical expert to resolve remaining open questions. In contrast, for an unexperienced operator it may be necessary to establish for example a video conference in order to efficiently resolve open questions. In addition, it may be necessary to send large amounts of image data to the remote medical expert.

The data set to be transmitted from the operator of the medical device to the remote medical expert also depends on the actual step in the workflow of the operator. For example, for an X-ray or ultrasound imaging device, the data set to be transmitted to the remote medical expert in the imaging preparation phase differs from the data set that needs to be transmitted in the image analysis phase. The communication unit may therefore be configured for determining different required transmission rates for the various steps of the operator's workflow. Accordingly, the communication unit of the medical device may also be configured for requesting and establishing different communication connections for different steps of the workflow.

In another exemplary embodiment, the communication unit of the medical device is configured for dividing the determined data set to be communicated to the remote medical expert into data packets. Moreover, the communication unit is configured for transmitting the data packets over one or more communication connections.

If one communication connection is not sufficient in order to provide the required transmission rate, then the communication unit of the medical device may automatically request and establish one or more other communication connections. The data to be transmitted to the remote medical expert is divided into data packets and these data packets may be transmitted over one or more communication connections. At the same time, it should be clear that the communication unit at the side of the remote medical expert has to be configured for receiving data packets over potentially more than one communication connection.

Since the communication connections may be based on different technologies, the communication connections may differ with respect to the length of data packets, the structure and length of header information, latency, error rates, routing techniques, security mechanisms and so forth. The communication units of the medical device and the remote medical expert are preferably configured for taking into account all these differences. For example, due to different latencies of communication connections, data packets may not be received by the communication unit at the side of the remote medical expert in their original order. Thus, the communication unit of the remote medical expert has to be configured for reassembling the data packets. In particular, the communication unit of the remote medical expert has to comprise buffers, which are large enough to compensate the latencies of the different communication connections. The communication unit of the medical device may include sequence numbers into the header information of the data packets so that the communication unit at the side of the remote medical expert can reassemble the data packets according to these sequence numbers.

Similarly, the communication unit at the side of the remote medical expert may be configured for dividing the data set to be transmitted to the operator of the medical device into data packets and for transmitting these data packets over one or more communication connections to the operator of the medical device. Thus, also the communication unit of the remote medical expert may be configured for transmitting data packets over one or more communication connections. At the same time, the communication unit of the medical device may be configured for receiving data packets over one or more communication connections and for sorting and combining the data packets received from the remote medical expert.

In another exemplary embodiment, the communication unit of the medical device is configured for supporting a set of different communication connections for transmitting data to the remote medical expert.

The communication unit of the medical device may support a plurality of wireless and wired communication connections. For example, the communication unit may be configured for supporting one or more communication connections over cellular communication networks using GSM, EDGE, WCDMA, CDMA2000, TDSCDMA, HSPA, HSPA+, LTE, LTE-advanced or other technologies. In addition, the communication unit may be configured for supporting Wi-Fi or satellite based connections. Moreover, the communication unit may be configured for requesting and utilizing communication repeater infrastructures based on communication drones or balloons. Other supported communication connections may be wired connections using for instance Ethernet as well as communication connections over networks owned by railway companies, airport operators and others. Last but not least, the communication unit may be configured for supporting also mesh and ad-hoc type of networks.

In another exemplary embodiment, the communication unit of the medical device is configured for providing a set of attributes for each communication connection and for requesting and establishing an optimum selection of communication connections for achieving the required transmission rate based on the attributes of the communication connections.

For each communication connection a set of attributes is stored by the communication unit. Such attributes may include information about access and priority rights. For example, one attribute may describe if the protocol of the communication connection allows to increase the priority of the data. Increasing the priority of the data may influence the scheduling of the data. In addition, it may result in a larger bandwidth assignment by the network by means of techniques such as carrier aggregation. Thus, increasing the priority of the data may result in higher transmission rates provided by the communication network. Other attributes of communication connections may be their local availability, billing information as well as information about the transmission properties. For example, one attribute may be the transmission rate that is achieved in 95% of the scenarios. Note that for wireless communication connections, the achievable transmission rate depends on a number of parameters including for example the bandwidth assigned by the network, the network congestion as well as channel properties such as slow and fast fading, multipath effects, Doppler shifts etc. For this reason, the transmission rates of wireless communication connections can vary widely and the information about a transmission rate that is achieved in most of the scenarios may be utilized in the optimum selection of communication connections.

The optimum selection of communication connections to achieve the required transmission rate may be performed by defining a suitable cost function to be minimized subject to the constraint that the transmission rate is greater than or equal to the required transmission rate. The cost function may depend on one or more of the attributes of the communication connections. Especially for wireless communication connections, the achieved transmission rates vary over time and are hardly predictable a priory. Consequently, the required transmission rate may not be achieved by the selected communication connections, since some of these connections may provide poor transmission rates or may not be available. In such a case, the communication unit may be configured for automatically requesting and establishing further communication connections to achieve the required transmission rate.

In another exemplary embodiment, the communication unit of the medical device is adapted as a modular communication platform, in which hardware modules can be inserted to extend the set of communication connections.

Thus, the communication unit of the medical device may comprise interfaces for connecting new hardware modules. These interfaces may be based for example on USB or RS232. The hardware modules may provide modem functionalities for certain communication technologies. Hence, by means of the interfaces it may be possible to flexibly extend the set of communication connections supported by the communication unit.

In another exemplary embodiment, the medical device further comprises a navigation receiver for receiving a navigation signal. Also, the communication unit is configured for selecting one or more communication connections taking into account the received navigation signal of the navigation receiver.

The medical device may comprise a navigation receiver such as a GPS, GLONASS, Galileo or Beidou receiver. The navigation receiver may be utilized to determine the location of the medical device. Alternatively, position information may be obtained using other positioning techniques such as radio beacons transmitted from known locations, internet geolocation such as Wi-Fi positioning, or positioning techniques utilizing cellular communication network infrastructures. The position information may be provided to the communication unit, which may have stored for each supported communication connection information about its local availability. Hence, by means of the position information from the navigation receiver, the communication unit of the medical device may determine those communication connections that are locally available. The optimum selection of communication connections to achieve the required transmission rate can then be performed by means of the communication unit taking into account only locally available connections.

In another exemplary embodiment, the attributes of the communication connections comprise databases specifying the local availability of the communication connections, and the medical device is configured for determining its position by means of the navigation receiver. Furthermore, the communication unit is configured for determining the locally available communication connections based on the databases specifying the local availability of communication connections and based on the position determined by the navigation receiver. In addition, the communication unit is configured for requesting and establishing one or more locally available communication connections to achieve the required transmission rate.

For example, for a cellular communication network, the data bases specifying the local availability of the communication connections may comprise position information of the base stations of the cellular network. Using the position information from the navigation receiver, the communication unit can determine its distance to the closest base station of the cellular network. If the medical device is located inside the coverage area of the base station, the communication unit may conclude that the cellular communication network is available.

For a communication connection using a drone or balloon based repeater infrastructure, the data bases may specify regions in which such drones or balloons are available or can be requested. Based on the position information from the navigation receiver, the communication unit can then determine if such a repeater infrastructure can be utilized at its current position. Alternatively, the communication unit may start a communication connection to some remote server to query if a drone or balloon based repeater infrastructure is available at its current position or if it can be established.

Alternatively, the communication unit of the medical device may be configured for determining the availability of communication connections by means of cell search procedures for cellular communication networks or by means of beacons that are transmitted for instance by Wi-Fi routers to indicate their presence.

In another exemplary embodiment, the communication unit of the medical device is configured for requesting a scheduling priority from a communication connection for the data to be transmitted to the remote medical expert.

Some communication networks allow to specify traffic prioritizations. The communication unit of the medical device may be configured for requesting a certain priority for the data to be transmitted to the remote medical expert. For example, if the communication unit of the medical device requests a high priority for the data to be transmitted to the remote medical expert, the communication network may assign more communication resources such as bandwidth, timeslots or codes to this connection. Moreover, the communication network may assign further carriers to this connection by means of carrier aggregation approaches. Hence, the communication connection may provide higher transmission rates and lower latencies for data that has a high priority.

In another exemplary embodiment, the communication unit of the medical device is configured for determining a required transmission rate and a required transmission time for transmitting data to the remote medical expert. In addition, the communication unit is configured for requesting and establishing one or more communication connections to achieve the required transmission rate for the required transmission time.

The medical device may determine a data set to be transmitted to the remote medical expert based on one or more current medical use conditions. Based on this information, the communication unit may assess the amount of data that needs to be transmitted to the remote medical expert. Taking into account the urgency level, the communication unit may compute the required time period and transmission rate for transmitting the data set to the remote medical expert. Hence, the communication unit may be configured for requesting and establishing one or more communication connections to achieve the required minimum transmission rate for the determined transmission time. In particular, the communication unit may be configured for signaling the communication network that the transmission rate is only required for the determined transmission time, so that the communication network can utilize this information in its resource allocation algorithms, in particular when prioritizing the medical data over data from other users.

In another exemplary embodiment, the communication unit of the medical device is configured for securely transmitting the determined data set to the remote medical expert using encryption and decryption.

Medical information of patients is typically highly sensitive private data that needs to be protected against misuse. For this reason, it is crucial that the data to be transmitted from the medical device to the remote medical expert is protected using state-of-the-art security techniques.

To achieve the required transmission rate, the communication unit of the medical device may request and establish more than one communication connections to the remote medical expert. In this case, the data to be transmitted to the remote medical expert may be divided into data packets, which may be transmitted over different communication connections. The communication unit may be configured for encrypting the data packets packet-by-packet. Alternatively, the communication unit may be configured for encrypting the data set to be transmitted to the remote medical expert at once.

In yet another exemplary embodiment, the medical device is an imaging device, preferably an X-ray imaging device, and the data set to be transmitted to the remote medical expert comprises imaging data, preferably X-ray imaging data.

X-ray and ultrasound imaging devices can be utilized for numerous diagnostic tasks. The user interface of such an imaging device may therefore be configured for querying input from the operator regarding his medical experience level in general, the diagnostic task to be performed, and the experience level of the operator with this diagnostic task. Moreover, the user interface may query input from the operator regarding his experience level with the imaging device. Based on the input provided by the operator, the imaging device may determine the data set that needs to be communicated to a remote medical expert to support the operator of the imaging device in resolving open questions. Especially ultrasound images are often hard to interpret, so the advice from a remote medical expert can be crucial for operators of ultrasound imaging devices. The communication unit of the imaging device may be configured for determining a transmission rate so that the operator of the imaging device and the remote medical expert can efficiently exchange information. To achieve this required transmission rate, the communication unit may request and establish one or more communication connections from a set of locally available communication connections.

For example, an experienced operator of an X-ray or ultrasound imaging device may need to exchange little information with the remote medical expert in order to clarify few open questions. In particular, the experienced operator of such an imaging device may transmit a preselected data set to the remote medical expert. In contrast, an operator with little experience with X-ray or ultrasound imaging may require substantial support from the remote medical expert. Therefore, the unexperienced operator of the imaging device may need to send large amounts of data to the remote medical expert. As a result, the communication unit of the imaging device may need to provide higher transmission rates in case of an unexperienced operator as compared to an experienced operator. Depending on the location, the higher transmission rates in case of an unexperienced operator may be achievable only by means of a combination of several communication connections. For this reason, the communication unit of the imaging device may be configured for requesting and establishing one or more communication connections to achieve the required transmission rate.

According to another aspect of the present invention, a method for facilitating remote medical support according to claim 10 is presented. In a merely illustrative embodiment, the method comprises the steps of querying an input regarding at least one current medical use condition of the medical device, receiving the input regarding the current medical use condition of the medical device, determining the required transmission rate for transmitting data from the medical device to a remote medical expert based on the received input regarding the current medical use condition of the medical device, and requesting and establishing one or more communication connections to achieve the required transmission rate.

The method is designed to improve the support of the operator of a medical device by a remote medical expert. Thereto, the operator of the medical device may be asked to provide information regarding the current medical use condition. More specifically, the operator may be asked to provide information about his medical experience level, his experience level with the medical device, the urgency level or the current use case of the medical device, i.e., the diagnostic task or treatment to be performed. The information provided by the operator may be utilized to assess the data set that needs to be transmitted from the communication unit of the medical device to the remote medical expert. In addition, a required transmission rate may be determined by the communication unit of the medical device to facilitate efficient communications between the operator of the medical device and the remote medical expert. The communication unit of the medical device may then request and establish one or more communication connections to the remote medical expert to achieve the required transmission rate.

Hence, the above method supports the operator of a medical device in determining the data set that actually needs to be transmitted to a remote medical expert to resolve open questions, avoiding superfluous data transmissions. In addition, by limiting the data sets to be transmitted to the remote medical expert to their relevant parts, and by requesting and establishing one or more communication connections to achieve the required transmission rate, it is ensured that the communication between the operator and the remote medical expert can be performed efficiently without unnecessary delays that may be critical in particular in emergency situations.

According to another aspect of the present invention, a program element for facilitating remote medical support according to claim 11 is presented. In a merely illustrative embodiment, the program element, when being executed by a processor, is adapted to carry out the steps of querying an input regarding at least one current medical use condition of a medical device, receiving the input regarding the current medical use condition of the medical device, determining a required transmission rate for transmitting data from the medical device to a remote medical expert based on the received input regarding the current medical use condition of the medical device, and requesting and establishing one or more communication connections to achieve the required transmission rate.

It should be clear to the person skilled in the art that the program element can be stored on various media including flash memory, hard disk drives or optical discs. Moreover, the program element may be executed on a number of computer processors.

In a merely illustrative embodiment, it may be seen as a gist of the present disclosure to provide a medical device that facilitates efficient communications between the operator of the medical device and a remote medical expert. Thereto, the medical device comprises a user interface, which is configured for querying information regarding at least one current medical use condition from the operator of the medical device. This information is utilized by the medical device to assess the data set that needs to be transmitted to the remote medical expert. The communication unit of the medical device is configured for determining a required transmission rate so that the data set can be transmitted to the remote medical expert without major delays. Hence, the required transmission rate is computed so that the operator of the medical device and the remote medical expert can efficiently communicate with each other. Furthermore, the communication unit of the medical device is configured for requesting and establishing one or more communication connections to the remote medical expert to achieve the required transmission rate. This may include dividing the data set into data packets and transmitting the data packets over different communication connections to the remote medical expert. Thus, efficient communications between the operator of the medical device and the remote medical expert are achieved by avoiding transmissions of superfluous data to the remote medical expert and by actively requesting and establishing one or more communication connections in order to achieve the required transmission rate. These and other features of the invention will become apparent from and elucidated with reference to the embodiments described hereinafter.

### BRIEF DESCRIPTION OF THE DRAWINGS

Fig. 1 shows key components of a medical device for facilitating remote medical support according to an exemplary embodiment of the present invention.
Fig. 2 shows a communication unit of the medical device for facilitating remote medical support according to an exemplary embodiment of the present invention.
Fig. 3 shows a communication scenario between a medical device and a remote medical expert according to an exemplary embodiment of the present invention.
Fig. 4 is a flow diagram illustrating the method for facilitating remote medical support.

In principle, identical parts are provided with the same reference symbols in the figures.

### DETAILED DESCRIPTION OF EMBODIMENTS

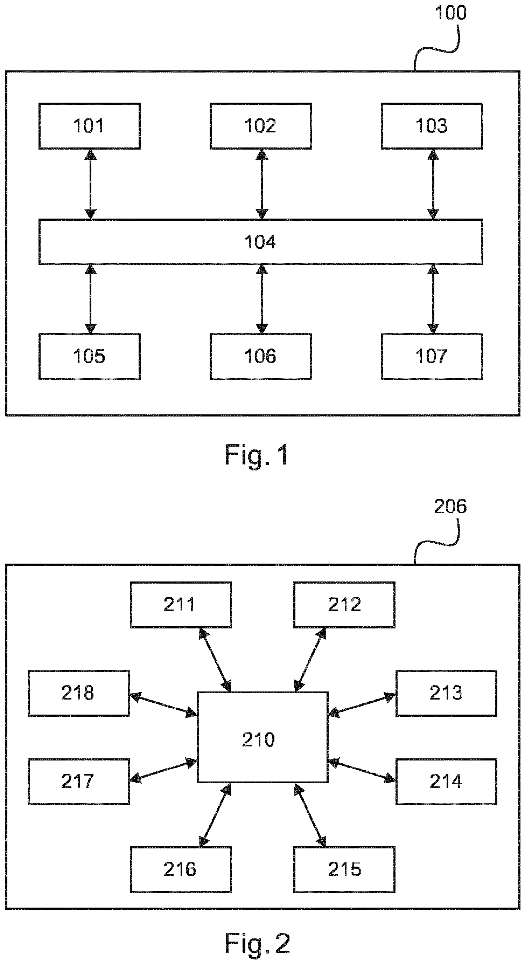

Figure 1 shows components of a medical device 100 for facilitating remote medical support according to an exemplary embodiment of the present invention. The medical device 100 comprises a touch display 101, a processing unit 102, a memory unit 103, a bus 104, a navigation receiver 105 and a communication unit 106. Other electronics of the medical device are represented by block 107.

The touch display 101 is used to realize a user interface, which is configured for querying and receiving an input from the operator of the medical device regarding the current medical use condition. This input regarding the medical use condition may comprise information about the medical experience level of the operator, the experience level of the operator with the medical device, the current use case of the medical device or the experience level of the operator with the current use case. Thereby, the use case may be a diagnostic task or medical treatment to be performed. Clearly, a number of alternative techniques other than a touch display may be used to realize the user interface.

The input regarding current medical use conditions provided by the operator is processed by the processing unit 102. Based on this input, the operator of the medical device 100 may be classified according to the required support level. That means, it may be determined how much support the operator may need from a remote medical expert. Moreover, a data set may be determined that needs to be communicated from the medical device to a remote medical expert in order to support the operator.

The processing unit 102 may be configured for determining required data processing steps based on the input regarding current medical use conditions. Moreover, the processing unit 102 may be configured for determining hardware resources that are necessary to perform the required data processing steps without undue delays. Such hardware resources may comprise graphics processing or storage units. In particular, the user interface of the medical device 100 may be configured for requesting that the operator provides additional hardware resources to prevent delays due to data processing bottlenecks.

Based on the required support level of the operator, the communication unit 106 determines a required transmission rate so that the operator of the medical device 100 can efficiently communicate with the remote medical expert. Subsequently, the communication unit 106 may request and establish one or more communication connections to achieve the required transmission rate. The communication unit 106 may be configured for keeping hardware modules of inactive communication technologies in a sleep mode to save battery power. Thus, the communication unit 106 may be configured for powering only hardware modules of active communication connections.

A navigation receiver 105 may provide position information to the communication unit 106. Assuming that the communication unit 106 comprises data bases specifying the local availability of communication connections, the communication unit can utilize the position information from the navigation receiver to determine the locally available communication connections. The data bases specifying the local availability of communication connections may comprise for example position information of the base stations for a cellular communication network.

Figure 2 shows the communication unit 206 of a medical device 100 for facilitating remote medical support according to an exemplary embodiment of the present invention. The communication unit comprises a connection management unit 210 as well as several modems 211 to 216 implementing different communication technologies. More specifically, the communication unit 206 comprises a first cellular network modem 211, a second cellular network modem 212, a Wi-Fi modem 213, an Ethernet modem 214, a satellite communications modem 215 as well as a modem 216 for repeater infrastructures using communication drones. The first and second cellular network modems 211 and 212 may each comprise a SIM card slot. Preferably, SIM cards from different network operators may be used to increase the diversity of wireless communication channels. That means, assuming that the medical device is located at a position where the communication network of the first SIM card provides poor transmission rates, the second SIM card may preferably be attached to some other network, which may provide better transmission rates at this location. Each modem may comprise a number of components such as RF circuitry, analog-to-digital conversion and sampling units, digital signal processors, power management units as well as hardware acceleration units for example for channel coding or encryption.

In addition, the communication unit 206 comprises a USB interface 217 and a connect card holder 218. The USB interface 217 and the connect card holder 218 may be utilized to connect hardware modules to the communication unit 206 in order to extend the set of supported communication technologies. That means, hardware modules inserted into the USB module 217 or the connect card holder 218 may allow requesting and establishing additional communication connections.

Based on the input regarding current medical use conditions, the connection management unit 210 determines the required transmission rate so that the operator of the medical device can be supported efficiently by the remote medical expert. Moreover, the connection management unit 210 requests and establishes one or more communication connections to achieve the required transmission rate. The connection management unit 210 may have stored for each communication connection a set of attributes. These attributes may comprise, amongst others, billing information, technical parameters such as typical transmission rates, or information regarding the local availability of communication connections. The local availability information and the position information provided by the navigation receiver 105 may be utilized by the connection management unit to determine the locally available communication connections. In case of problems with one or more communication connections, the connection management unit 210 may automatically request and establish further communication connections to achieve the required transmission rate.

The connection management unit 210 may split the data set to be transmitted from the medical device to the remote medical expert into data packets. These data packets may be transmitted over one or more communication connections to the remote medical expert. Hence, the communication unit at the side of the remote medical expert should be configured for supporting one or more communication connections and for reassembling the data packets received from the medical device.

Note that similar principles as for the communication from the medical device 100 to the remote medical expert may be applied for the communication in the reverse direction from the remote medical expert to the medical device. In particular, the connection management unit 210 may be configured for determining required transmission rates for communications to and from a remote medical expert. Moreover, the connection management unit 210 may be configured for requesting and establishing communication connections so that the required transmission rates to and from the remote medical expert are met. In addition, the communication units of both the medical device and the remote medical expert may be configured for transmitting and receiving data packets over one or more communication connections.

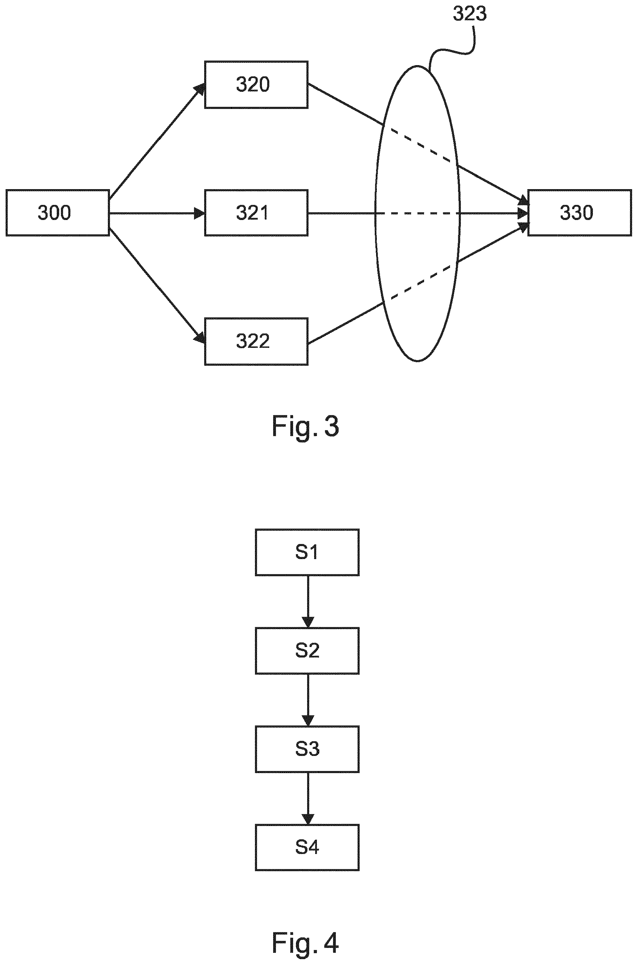

Figure 3 illustrates a communication scenario from a medical device 300 to a remote medical expert 330. The medical device 300 may be located in a rural area, whereas the remote medical expert may be located in a distant hospital. The communication unit of the medical device 300 has established three communication connections 320, 321 and 322 with the remote medical expert 330. More specifically, 320 represents a connection over a mobile radio access network. Moreover, 321 represents a Wi-Fi connection, and 322 represents a communication connection using a repeater infrastructure based on communication drones. The data packets transmitted over the communication connections 320, 321 and 322 are forwarded through communication networks 323 to the remote medical expert 330.

Figure 4 illustrates the method for facilitating remote medical support for an operator of a medical device. In step S1, at least one input regarding current medical use conditions is queried. Medical use conditions can be, amongst others, the medical experience level of the operator of the medical device, the experience level of the operator with the specific medical device, the use case of the medical device or the experience of the operator with the particular use case, that means, the experience of the operator with the diagnostic task or medical treatment to be performed. In step S2, the at least one input regarding current medical use conditions is received. Steps S1 and S2 may be realized by means of a user interface. In step S3, a required transmission rate for transmitting data from the medical device to the remote medical expert is determined. Thereto, a data set may be determined that is to be transmitted from the medical device to the remote medical expert. This data set may depend on the input regarding medical use conditions provided by the operator. In step S4, one or more communication connections are requested and established to achieve the required transmission rate. Thereby, the position information from the navigation receiver may be utilized to determine those communication connections that are available at the current location.

Other variations to the disclosed embodiments can be understood and effected by those skilled in the art in practicing the claimed invention, from the study of the drawings, the disclosure, and the appended claims. In the claims the word "comprising" does not exclude other elements or steps and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope of the claims.

Where an indefinite or definite article is used when referring to a singular noun, e.g. "a", "an" or "the", this includes a plurality of that noun unless something else is specifically stated. The terms "about" or "approximately" in the context of the present invention denote an interval of accuracy that the person skilled in the art will understand to still ensure the technical effect of the feature in question. The term typically indicates deviation from the indicated numerical value of ±20 %, preferably ±15 %, more preferably ±10 %, and even more preferably ±5 %.

## Claims

1. Medical device (100) for facilitating remote medical support, the device comprising:
- a user interface, and
- a communication unit (106),
wherein the user interface is configured to query an input regarding at least one current medical use condition of the medical device,
wherein the user interface is configured to receive the input regarding the current medical use condition of the medical device, wherein the input comprises the current use case of the medical device and the experience level of the operator with the current use case, and wherein the use case is a diagnostic task or medical treatment to be performed,
wherein the medical device is configured to utilize the input to determine a support level that is needed for the operator, wherein the support level relates to the amount of support the operator requires from a remote medical expert to perform a plurality of steps in the current use case,
wherein the medical device is configured to utilize the support level to determine a plurality of data sets that need to be communicated to the remote medical expert, and wherein a different data set is determined for each step of the plurality of steps,
wherein the communication unit is configured to determine different required transmission rates for transmitting each data set of the plurality of data sets from the medical device to the remote medical expert,
wherein the communication unit is configured to request and establish a plurality of communication connections to achieve the different required transmission rates; and
wherein when a communication connection is not sufficient to provide the required transmission for a specific data set then the communication unit (106) is configured to divide the specific data set into data packets, and the communication unit is configured to request and establish one or more other communication connections, and the communication unit is configured for transmitting the data packets over the one or more other communication connections.

2. Medical device (100) according to claim 1,
wherein the communication unit (106) is configured for providing a set of attributes for each communication connection, and
wherein the communication unit is configured for requesting and establishing an optimum selection of communication connections for achieving the required transmission rate based on the attributes of the communication connections.

3. Medical device (100) according to one of claims 1 or 2,
wherein the communication unit (106) is adapted as a modular communication platform, in which hardware modules can be inserted to extend the set of communication connections.

4. Medical device (100) according to any of claims 1 to 3,
further comprising a navigation receiver for receiving a navigation signal, and
wherein the communication unit (106) is configured for selecting one or more communication connections taking into account the received navigation signal of the navigation receiver.

5. Medical device (100) according to claim 4,
wherein the attributes of the communication connections comprise databases specifying the local availability of the communication connections,
wherein the medical device is configured for determining its position by means of the navigation receiver,
wherein the communication unit (106) is configured for determining the locally available communication connections based on the databases specifying the local availability of communication connections and based on the position determined by the navigation receiver, and
wherein the communication unit is configured for requesting and establishing one or more locally available communication connections to achieve the required transmission rate.

6. Medical device (300) according to any of the preceding claims,
wherein the communication unit (106) is configured for requesting a scheduling priority from a communication connection for the data to be transmitted to the remote medical expert (330).

7. Medical device (300) according to any of the preceding claims,
wherein the communication unit (106) is configured for determining a required transmission rate and a required transmission time for transmitting data to the remote medical expert (330), and
wherein the communication unit is configured for requesting and establishing one or more communication connections to achieve the required transmission rate for the required transmission time.

8. Medical device (300) according to any of the preceding claims,
wherein the communication unit (106) is configured for securely transmitting the determined data set to the remote medical expert (330) using encryption and decryption.

9. Medical device (300) according to any of the preceding claims,
wherein the medical device is an imaging device, preferably an X-ray or ultrasound imaging device, and
wherein the data set to be transmitted to the remote medical expert (330) comprises imaging data, preferably X-ray or ultrasound imaging data.

10. A computer-implemented method for facilitating remote medical support, the method comprising the steps:
- querying an input regarding at least one current medical use condition of a medical device (S 1),
- receiving the input regarding the current medical use condition of the medical device (S2), wherein the input comprises the current use case of the medical device and the experience level of the operator with the current use case, and wherein the use case is a diagnostic task or medical treatment to be performed,
- utilizing the input to determine a support level that is needed for the operator, wherein the support level relates to the amount of support the operator requires from a remote medical expert to perform a plurality of steps in the current use case,
- utilizing the support level to determine a plurality of data sets that need to be communicated to the remote medical expert, and wherein a different data set is determined for each step of the plurality of steps,
- determining different required transmission rates for transmitting each data set of the plurality of data sets from the medical device to the remote medical expert (S3),
- requesting and establishing a plurality ofcommunication connections to achieve the different required transmission rate (S4), and
- when a communication connection is not sufficient to provide the required transmission for a specific data set, then dividing the specific data set into data packets, and requesting and establishing one or more other communication connections and transmitting the data packets over the one or more other communication connections.

11. A program element for facilitating remote medical support, which program element, when being executed by a processor, is adapted to carry out the method of claim 10.

## Patentansprüche

1. Medizinische Vorrichtung (100) zur Erleichterung der medizinischen Fernunterstützung, wobei die Vorrichtung Folgendes umfasst:
- eine Benutzerschnittstelle und
- eine Kommunikationseinheit (106),
wobei die Benutzerschnittstelle konfiguriert ist, um eine Eingabe bezüglich mindestens eines aktuellen medizinischen Verwendungszustands der medizinischen Vorrichtung abzufragen,
wobei die Benutzerschnittstelle konfiguriert ist, um die Eingabe bezüglich des aktuellen medizinischen Verwendungszustands der medizinischen Vorrichtung zu empfangen, wobei die Eingabe den aktuellen Anwendungsfall der medizinischen Vorrichtung und den Erfahrungsgrad des Bedieners mit dem aktuellen Anwendungsfall umfasst, und wobei der Anwendungsfall eine durchzuführende Diagnoseaufgabe oder medizinische Behandlung ist,
wobei die medizinische Vorrichtung so konfiguriert ist, dass es die Eingabe nutzt, um ein Unterstützungsniveau zu bestimmen, das für den Bediener benötigt wird, wobei sich das Unterstützungsniveau auf das Ausmaß der Unterstützung bezieht, die der Bediener von einem entfernten medizinischen Experten benötigt, um eine Vielzahl von Schritten im aktuellen Anwendungsfall durchzuführen,
wobei die medizinische Vorrichtung so konfiguriert ist, dass es das Unterstützungsniveau nutzt, um eine Vielzahl von Datensätzen zu bestimmen, die an den entfernten medizinischen Experten kommuniziert werden müssen, und wobei für jeden Schritt der Vielzahl von Schritten ein anderer Datensatz bestimmt wird,
wobei die Kommunikationseinheit konfiguriert ist, um unterschiedliche erforderliche Übertragungsraten zum Übertragen jedes Datensatzes der Vielzahl von Datensätzen von der medizinischen Vorrichtung an den entfernten medizinischen Experten zu ermitteln,
wobei die Kommunikationseinheit konfiguriert ist, um mehrere Kommunikationsverbindungen anzufordern und aufzubauen, um die unterschiedlichen erforderlichen Übertragungsraten zu erreichen; und
wobei, wenn eine Kommunikationsverbindung nicht ausreicht, um die erforderliche Übertragung für einen bestimmten Datensatz bereitzustellen, die Kommunikationseinheit (106) konfiguriert ist, um den spezifischen Datensatz in Datenpakete aufzuteilen, und die Kommunikationseinheit konfiguriert ist, um eine oder mehrere andere Kommunikationsverbindungen anzufordern und aufzubauen, und die Kommunikationseinheit konfiguriert ist, um die Datenpakete über die eine oder mehreren anderen Kommunikationsverbindungen zu übertragen.

2. Medizinische Vorrichtung (100) nach Anspruch 1,
wobei die Kommunikationseinheit (106) zum Bereitstellen eines Satzes von Attributen für jede Kommunikationsverbindung konfiguriert ist, und
wobei die Kommunikationseinheit zum Anfordern und Aufbauen einer optimalen Auswahl von Kommunikationsverbindungen zum Erreichen der erforderlichen Übertragungsrate basierend auf den Attributen der Kommunikationsverbindungen konfiguriert ist.

3. Medizinische Vorrichtung (100) nach einem der Ansprüche 1 oder 2,
wobei die Kommunikationseinheit (106) als modulare Kommunikationsplattform ausgelegt ist, in die Hardwaremodule eingefügt werden können, um den Satz von Kommunikationsverbindungen zu erweitern.

4. Medizinische Vorrichtung (100) nach einem der Ansprüche 1 bis 3, weiterhin umfassend einen Navigationsempfänger zum Empfangen eines Navigationssignals und
wobei die Kommunikationseinheit (106) zur Auswahl einer oder mehrerer Kommunikationsverbindungen unter Berücksichtigung des empfangenen Navigationssignals des Navigationsempfängers konfiguriert ist.

5. Medizinische Vorrichtung (100) nach Anspruch 4,
wobei die Attribute der Kommunikationsverbindungen Datenbanken umfassen, die die lokale Verfügbarkeit der Kommunikationsverbindungen angeben,
wobei die medizinische Vorrichtung zur Bestimmung seiner Position mittels des Navigationsempfängers konfiguriert ist,
wobei die Kommunikationseinheit (106) konfiguriert ist, um die lokal verfügbaren Kommunikationsverbindungen basierend auf den Datenbanken, die die lokale Verfügbarkeit von Kommunikationsverbindungen angeben, und basierend auf der vom Navigationsempfänger ermittelten Position zu ermitteln, und
wobei die Kommunikationseinheit konfiguriert ist, um eine oder mehrere lokal verfügbare Kommunikationsverbindungen anzufordern und aufzubauen, um die erforderliche Übertragungsrate zu erreichen.

6. Medizinische Vorrichtung (300) nach einem der vorhergehenden Ansprüche,
wobei die Kommunikationseinheit (106) zum Anfordern einer Planungspriorität von einer Kommunikationsverbindung für die an den entfernten medizinischen Experten (330) zu übertragenden Daten konfiguriert ist.

7. Medizinische Vorrichtung (300) nach einem der vorhergehenden Ansprüche,
wobei die Kommunikationseinheit (106) zum Bestimmen einer erforderlichen Übertragungsrate und einer erforderlichen Übertragungszeit zum Übertragen von Daten an den entfernten medizinischen Experten (330) konfiguriert ist, und
wobei die Kommunikationseinheit konfiguriert ist, um eine oder mehrere Kommunikationsverbindungen anzufordern und aufzubauen, um die erforderliche Übertragungsrate für die erforderliche Übertragungszeit zu erreichen.

8. Medizinische Vorrichtung (300) nach einem der vorhergehenden Ansprüche,
wobei die Kommunikationseinheit (106) zum sicheren Übertragen des ermittelten Datensatzes an den entfernten medizinischen Experten (330) unter Verwendung von Verschlüsselung und Entschlüsselung konfiguriert ist.

9. Medizinische Vorrichtung (300) nach einem der vorhergehenden Ansprüche,
wobei die medizinische Vorrichtung eine Bildgebungsvorrichtung, vorzugsweise eine Röntgen- oder Ultraschallbildgebungsvorrichtung, ist, und
wobei der an den entfernten medizinischen Experten (330) zu übertragende Datensatz Bilddaten, vorzugsweise Röntgen- oder Ultraschallbilddaten, umfasst.

10. Computerimplementiertes Verfahren für Erleichterung der medizinischen Fernunterstützung, wobei das Verfahren die Schritte umfasst von:
- Abfragen einer Eingabe bezüglich mindestens eines aktuellen medizinischen Verwendungszustands einer medizinischen Vorrichtung (S1),
- Empfangen der Eingabe bezüglich des aktuellen medizinischen Verwendungszustands der medizinischen Vorrichtung (S2), wobei die Eingabe den aktuellen Anwendungsfall der medizinischen Vorrichtung und den Erfahrungsgrad des Bedieners mit dem aktuellen Anwendungsfall umfasst, und wobei der Anwendungsfall eine durchzuführende Diagnoseaufgabe oder medizinische Behandlung ist,
- Verwenden der Eingabe, um ein Unterstützungsniveau zu bestimmen, das für den Bediener benötigt wird, wobei sich das Unterstützungsniveau auf das Ausmaß der Unterstützung bezieht, das der Bediener von einem entfernten medizinischen Experten benötigt, um eine Vielzahl von Schritten im aktuellen Anwendungsfall durchzuführen,
- Verwenden des Unterstützungsniveaus, um eine Vielzahl von Datensätzen zu bestimmen, die an den entfernten medizinischen Experten übermittelt werden müssen, und wobei ein anderer Datensatz bestimmt wird für jeden Schritt der Vielzahl von Schritten,
- Bestimmen unterschiedlicher erforderlicher Übertragungsraten zum Übertragen jedes Datensatzes der Vielzahl von Datensätzen von der medizinischen Vorrichtung zum entfernten medizinischen Experten (S3),
- Anfordern und Herstellen einer Vielzahl von Kommunikationsverbindungen, um die unterschiedlichen erforderlichen Übertragungsraten zu erreichen (S4), und
- wenn eine Kommunikationsverbindung nicht ausreicht, um die erforderliche Übertragung für einen bestimmten Datensatz bereitzustellen, dann Aufteilen des bestimmten Datensatzes in Datenpakete und Anfordern und Einrichten einer oder mehrerer anderer Kommunikationsverbindungen und Übertragen der Datenpakete über die eine oder mehrere andere Kommunikation Verbindungen.

11. Programmelement zur Erleichterung der medizinischen Fernunterstützung, wobei das Programmelement, wenn es von einem Prozessor ausgeführt wird, geeignet ist, um das Verfahren nach Anspruch 10 auszuführen.

## Revendications

1. Dispositif médical (100) pour faciliter l'assistance médicale à distance, le dispositif comprenant :
- une interface utilisateur et
- une unité de communication (106),
où l'interface utilisateur est configurée pour interroger une entrée concernant au moins une condition d'utilisation médicale actuelle du dispositif médical,
où l'interface utilisateur est configurée pour recevoir l'entrée concernant la condition d'utilisation médicale actuelle du dispositif médical, où l'entrée comprend le cas d'utilisation actuel du dispositif médical et le niveau d'expérience de l'opérateur avec le cas d'utilisation actuel, et où le cas d'utilisation est une tâche diagnostique ou un traitement médical à effectuer,
où le dispositif médical est configuré pour utiliser l'entrée pour déterminer un niveau d'assistance qui est nécessaire pour l'opérateur, où le niveau d'assistance est lié à l'ampleur de l'assistance dont l'opérateur a besoin d'un expert médical à distance pour effectuer une pluralité d'étapes dans le cas d'utilisation actuel,
où le dispositif médical est configuré pour utiliser le niveau d'assistance pour déterminer une pluralité d'ensembles de données qui doivent être communiqués à l'expert médical à distance, et où un ensemble de données différentes est déterminé pour chaque étape de la pluralité d'étapes,
où l'unité de communication est configurée pour déterminer différents débits de transmission requis pour transmettre chaque ensemble de données de la pluralité d'ensembles de données du dispositif médical à l'expert médical à distance,
où l'unité de communication est configurée pour demander et établir une pluralité de connexions de communication pour atteindre les différents débits de transmission requis; et
où, lorsqu'une connexion de communication n'est pas suffisante pour fournir la transmission requise pour un ensemble de données spécifique, l'unité de communication (106) est alors configurée pour diviser l'ensemble de données spécifique en paquets de données, et l'unité de communication est configurée pour demander et établir une ou plusieurs autres connexions de communication, et l'unité de communication est configurée pour transmettre les paquets de données sur l'une ou plusieurs autres connexions de communication.

2. Dispositif médical (100) selon la revendication 1,
dans lequel l'unité de communication (106) est configurée pour fournir un ensemble d'attributs pour chaque connexion de communication, et
dans lequel l'unité de communication est configurée pour demander et établir une sélection optimale de connexions de communication pour atteindre le débit de transmission requis sur la base des attributs des connexions de communication.

3. Dispositif médical (100) selon l'une des revendications 1 ou 2,
dans lequel l'unité de communication (106) est adaptée en tant que plate-forme de communication modulaire, dans laquelle des modules matériels peuvent être insérés pour étendre l'ensemble de connexions de communication.

4. Dispositif médical (100) selon l'une quelconque des revendications 1 à 3,
comprenant en outre un récepteur de navigation pour recevoir un signal de navigation, et
dans lequel l'unité de communication (106) est configurée pour sélectionner une ou plusieurs connexions de communication en tenant compte du signal de navigation reçu du récepteur de navigation.

5. Dispositif médical (100) selon la revendication 4,
dans lequel les attributs des connexions de communication comprennent des bases de données spécifiant la disponibilité locale des connexions de communication,
dans lequel le dispositif médical est configuré pour déterminer sa position au moyen du récepteur de navigation,
dans lequel l'unité de communication (106) est configurée pour déterminer les connexions de communication disponibles localement sur la base des bases de données spécifiant la disponibilité locale des connexions de communication et sur la base de la position déterminée par le récepteur de navigation, et
dans lequel l'unité de communication est configurée pour demander et établir une ou plusieurs connexions de communication disponibles localement afin d'atteindre le débit de transmission requis.

6. Dispositif médical (300) selon l'une quelconque des revendications précédentes,
dans lequel l'unité de communication (106) est configurée pour demander une priorité de planification à partir d'une connexion de communication pour les données à transmettre à l'expert médical à distance (330) .

7. Dispositif médical (300) selon l'une quelconque des revendications précédentes,
dans lequel l'unité de communication (106) est configurée pour déterminer un débit de transmission requis et un temps de transmission requis pour transmettre des données à l'expert médical à distance (330), et
dans lequel l'unité de communication est configurée pour demander et établir une ou plusieurs connexions de communication afin d'atteindre le débit de transmission requis pendant la durée de transmission requise.

8. Dispositif médical (300) selon l'une quelconque des revendications précédentes,
dans lequel l'unité de communication (106) est configurée pour transmettre de manière sécurisée l'ensemble de données déterminé à l'expert médical à distance (330) à l'aide du cryptage et du décryptage.

9. Dispositif médical (300) selon l'une quelconque des revendications précédentes,
dans lequel le dispositif médical est un dispositif d'imagerie, de préférence un dispositif d'imagerie à rayons X ou à ultrasons, et
dans lequel l'ensemble de données à transmettre à l'expert médical à distance (330) comprend des données d'imagerie, de préférence des données d'imagerie à rayons X ou à ultrasons.

10. Procédé implémenté par ordinateur
pour faciliter l'assistance médicale à distance, le procédé comprenant les étapes consistant à:
- interroger une entrée concernant au moins une condition d'utilisation médicale actuelle d'un dispositif médical (S1),
- recevoir l'entrée concernant la condition d'utilisation médicale actuelle du dispositif médical (S2), où l'entrée comprend le cas d'utilisation actuel du dispositif médical et le niveau d'expérience de l'opérateur avec le cas d'utilisation actuel, et où le cas d'utilisation est une tâche diagnostique ou un traitement médical à effectuer,
- utiliser l'entrée pour déterminer un niveau d'assistance qui est nécessaire pour l'opérateur, où le niveau d'assistance est lié à l'ampleur de l'assistance dont l'opérateur a besoin d'un expert médical à distance pour effectuer une pluralité d'étapes dans le cas d'utilisation actuel,
- utiliser le niveau d'assistance pour déterminer une pluralité d'ensembles de données qui doivent être communiqués à l'expert médical à distance, et où un ensemble de données différentes est déterminé pour chaque étape de la pluralité d'étapes,
- déterminer différents débits de transmission requis pour transmettre chaque ensemble de données de la pluralité d'ensembles de données du dispositif médical à l'expert médical à distance (S3),
- demander et établir une pluralité de connexions de communication pour atteindre les différents débits de transmission requis (S4), et
- lorsqu'une connexion de communication n'est pas suffisante pour fournir la transmission requise pour un ensemble de données spécifique, alors diviser l'ensemble de données spécifique en paquets de données, et demander et établir une ou plusieurs autres connexions de communication et transmettre les paquets de données sur l'une ou plusieurs autres connexions de communication.

11. Élément de programme pour faciliter l'assistance médical à distance, l'élément de programme, lorsqu'il est exécuté par un processeur, étant adapté pour mettre en oeuvre le procédé selon la revendication 10.
